Europäisches Patentamt

European Patent Office

Office européen des brevets

(11) Veröffentlichungsnummer : **0 126 349**
**B1**

(12) **EUROPÄISCHE PATENTSCHRIFT**

(45) Veröffentlichungstag der Patentschrift :
20.08.86

(51) Int. Cl.⁴ : **C 07 C 69/533, C 07 C 67/333**

(21) Anmeldenummer : 84104989.3

(22) Anmeldetag : 03.05.84

(54) **Verfahren zur Herstellung von 4-Pentensäureestern.**

(30) Priorität : 11.05.83 DE 3317163

(43) Veröffentlichungstag der Anmeldung :
28.11.84 Patentblatt 84/48

(45) Bekanntmachung des Hinweises auf die Patenterteilung : 20.08.86 Patentblatt 86/34

(84) Benannte Vertragsstaaten :
BE CH DE FR GB IT LI NL

(56) Entgegenhaltungen :
US-A- 3 161 672
US-A- 4 327 225

(73) Patentinhaber : BASF Aktiengesellschaft
Carl-Bosch-Strasse 38
D-6700 Ludwigshafen (DE)

(72) Erfinder : Schneider, Heinz-Walter, Dr.
Bruesseler Ring 43
D-6700 Ludwigshafen (DE)
Erfinder : Kummer, Rudolf, Dr.
Kreuzstrasse 6
D-6710 Frankenthal (DE)

EP 0 126 349 B1

## Beschreibung

Bei der Herstellung von Pentensäureestern durch Umsetzung von Butadien mit Kohlenmonoxyd und Alkoholen in Gegenwart von Metallcarbonylkatalysatoren, wie es zum Beispiel in der DE-OS-30 40 432 beschrieben wird, erhält man erhebliche Mengen an isomeren Pentensäureestern. Für weitere Umsetzungen z. B. zum Herstellen von δ-Formylvaleriansäureestern durch Hydroformylierung von Pentensäureester wird jedoch 4-Pentensäureester als Ausgangsverbindung bevorzugt. Es wurde deshalb schon versucht 4-Pentensäureester durch Isomerierung von isomeren Pentensäureester zu erhalten. Wie aus Bull. of the Chem. Soc. of Japan Band 46, Seite 528 bekannt ist, erhält man durch Isomerisieren von 3-Pentensäuremethylester in Gegenwart von Cobaltcarbonylen jedoch vorwiegend 2-Pentensäuremethylester. Nach einer anderen in Tetrahedron Vol. 28, Seiten 5769-77 (1972) beschriebenen Arbeitsweise gelingt es zwar in Gegenwart eines Komplexes aus Rhodiumtriphenylphosphin und Zinnchlorid das Isomerengleichgewicht so zu verschieben, daß 4-Pentensäureester erhalten werden. Der dort verwendete Katalysator wird jedoch innerhalb weniger Stunden inaktiv.

Bei der Isomerisierung des Pentensäureesters liegen nach Erreichen des thermodynamischen Gleichgewichts fünf Isomere nämlich 4-Pentensäureester, cis- und trans-3-Pentensäureester sowie cis- und trans-2-Pentensäureester vor, wobei das Gleichgewicht stark nach der Seite des trans-2-Pentenesters verschoben ist und weniger als 3 % 4-Pentensäureester enthalten sind.

Es war deshalb die technische Aufgabe gestellt, ein Verfahren zur Herstellung von 4-Pentenester aus isomeren Pentenestern zur Verfügung zu stellen, bei dem die Katalysatoren über längere Zeit ihre Aktivität behalten und zudem die lineare Verschiebung der Doppelbindung zum 4-Pentenester bevorzugt verläuft und zudem möglichst wenig des schwer abtrennbaren cis-2-Pentenesters gebildet wird.

Diese technische Aufgabe wird gelöst in einem Verfahren zur Herstellung von 4-Pentenester durch Isomerisierung von isomeren Pentensäureestern bei erhöhter Temperatur in Gegenwart von Katalysatoren, wobei man Pentenester mit sauren Ionenaustauschern oder sauren Zeolithen, die ein Gehalt an Edelmetallen der achten Gruppe des periodischen Systems haben, behandelt und 4-Pentenester aus dem Reaktionsgemisch abdestilliert.

Das neue Verfahren hat den Vorteil, daß die verwendeten Katalysatoren eine lange Lebensdauer haben, d. h. für eine Vielzahl von Recyclisierungen eine ausreichende Aktivität beibehalten und leicht reaktiviert sind. Besonders hat das neue Verfahren den Vorteil, daß bevorzugt eine lineare Verschiebung der Doppelbindung zu 4-Pentenester eintritt, wobei die Konzentration des 4-Pentensäureesters erheblich über derjenigen liegt, die dem thermodynamischen Gleichgewicht entspricht.

Vorteilhaft geht man von isomeren Pentensäureestern z. B. 2 oder 3-Pentensäureestern aus, die sich von Alkoholen mit bis zu 12 Kohlenstoffatomen herleiten. Besonders bevorzugt verwendet man isomere Pentensäurealkylester insbesondere von Alkoholen mit bis zu 4 Kohlenstoffatomen. Geeignete 3-Pentensäureester sind beispielsweise 3-Pentensäuremethylester, 3-Pentensäureethylester, 3-Pentensäurepropylester, 3-Pentensäurebutylester oder oder 2-Pentensäuremethylester, 2-Pentensäureethylester sowie 2-Pentensäurepropylester.

Als Katalysatoren verwendet man saure Ionenaustauscher oder saure Zeolithe. Bevorzugt werden stark saure Ionenaustauscher z. B. vernetzte Polystyrole mit sauren Gruppen, insbesondere Sulfonsäuregruppen verwendet. Besonders bewährt haben sich Styrol-Divinylbenzol-Copolymerisate mit Sulfonsäuregruppen. Bevorzugte saure Zeolithe sind A-, X- oder Y-Zeolithe in der H-Form, d. h. in seiner sauren Form.

Die sauren Ionenaustauscher oder sauren Zeolithe haben einen Gehalt an Edelmetallen der achten Gruppe des periodischen Systems. Besonders bevorzugt sind Palladium, Ruthenium oder Rhodium insbesondere jedoch Palladium. Der Metallgehalt beträgt vorzugsweise 0,01 bis 1 Gew.- %. Die Katalysatoren können fest angeordnet oder als Suspension verwendet werden.

Die Verweilzeit der isomeren Pentensäureester am Katalysator hängt von der katalytischen Aktivität des Katalysators und der Isomerisierungsgeschwindigkeit ab. Sie beträgt in der Regel von 0,01 bis 1 Stunden. Vorteilhaft wendet man je kg isomere Pentensäureester 0,01 bis 0,3 kg Katalysator an.

Die Temperatur richtet sich im wesentlichen nach dem Siedepunkt des herzustellenden 4-Pentensäureesters. Vorteilhaft hält man Temperaturen von 100 bis 150 °C ein. Bei der Verwendung von sauren Ionenaustauschern auf Polystyrolbasis haben sich Temperaturen von 110 bis 140 °C bewährt. Die Isomerisierung führt man in der Regel unter Normaldruck durch. Je nach Art des verwendeten Esters können jedoch auch verminderter Druck oder schwach erhöhtem Druck z. B. bis zu 3 bar angewandt werden.

Nachdem sich ausreichend 4-Pentensäureester gebildet hat, wird dieser aus dem Isomerengemisch abdestilliert und das verbleibende Isomerengemisch wiederum mit dem Katalysator in Berührung gebracht. Es wird somit 4-Pentensäureester aus dem Isomerisierungsgemisch fortlaufend abdestilliert.

Die Behandlung mit dem Katalysator kann absatzweise erfolgen, wird jedoch vorzugsweise kontinuierlich durchgeführt. Das verbleibende Isomerengemisch wird zweckmäßig wieder in die Isomerisierung zurückgeführt.

4-Pentensäureester eignen sich zur Herstellung von δ-Formylvaleriansäureester, einem Vorprodukt für die Herstellung von von ε-Caprolactam, Hexandiol oder Adipinsäure.

Das Verfahren sei an folgenden Beispielen veranschaulicht :

Beispiele

Beispiel 1

In einem Glaskolben werden 100 g eines Gemisches aus 70 Gew.- % 3-trans- und 30 Gew.- % 3-cis-Pentensäuremethylester, wie es bei der Carbonylierung von Butadien-1,3 erhalten wird, mit 10 g Katalysator von Typ Y-Zeolith beladen mit 0,5 Gew.- % Palladium bei 135 °C gerührt. Nach 6 Minuten Reaktionszeit erhält man ein Reaktionsgemisch, das sich aus 8 Gew.- % 4-, 0,1 Gew.- % 2-cis-, 64,3 Gew.- % 3-trans-, 27,4 Gew.- % 3-cis- und 0,2 % 2-trans-pentensäureester zusammensetzt.

Die Katalysatorproduktivität für 4-Pentensäuremethylester beträgt 1 600 g pro g Pd und Stunde.

Nach Abtrennen des Katalysators wird das Reaktionsgemisch in einer Kolonne (Trennstufenzahl 100, Rücklauf 50) aufdestilliert. Dabei werden bei einer Kopftemperatur von 126 °C, 8,2 g 4-Pentensäuremethylester mit einer Reinheit 95 % abgenommen. Der Destillationssumpf (91,8 g) wird nach Ergänzen der abdestillierten Menge (8,2 g) mit frischem Pentensäureestergemisch der anfangs genannten Zusammensetzung erneut zur Isomerisierung eingesetzt.

Beispiel 2

Man verfährt analog Beispiel 1, verwendet jedoch einen mit 0,2 % Palladium beladenen sauren Ionenaustauscher aus vernetztem Polystyrol. Nach 18 Minuten Reaktionszeit erhält man ein Reaktionsgemisch mit 8,0 Gew.- % 4-Pentensäureester.

Die Katalysatorproduktivität für 4-Pentensäureester beträgt 1 330 pro g Pd und Stunde.

Beispiel 3

Man verfährt analog Beispiel 1, verwendet jedoch einen mit 0,7 % Rhodium beladenen sauren Ionenaustauscher. Nach 25 Minuten Reaktionszeit erhält man ein Reaktionsgemisch mit 8 Gew.- % 4-Pentensäureester.

Die Katalysatorproduktivität für 4-Pentensäureester beträgt 271 g pro g Rh und Stunde.

Beispiel 4

Man verfährt analog Beispiel 1, verwendet jedoch einen mit 0,4 % Ruthenium beladenen sauren Ionenaustauscher. Nach 96 Minuten erhält man ein Reaktionsgemisch mit 8 % 4-Pentensäureester.

Die Katalysatorproduktivität für 4-Pentensäureester beträgt 125 g pro g Ru und Stunde.

Beispiel 5

In einem Glaskolben werden 100 g eines Gemisches aus 70 Gew.- % 3-trans- und 30 Gew.- % 3-cis-Pentensäuremethylester, wie es bei der Carbonylierung von Butadien-1,3 erhalten wird, mit 10 g Katalysator vom Typ Y-Zeolith beladen mit 0,5 Gew.- % Palladium bei 135 °C gerührt. Nach 6 Minuten Reaktionszeit erhält man ein Reaktionsgemisch, das sich aus 8 Gew.- % 4-, 0,1 Gew.- % 2-cis-, 64,3 Gew.- % 3-trans-, 27,4 Gew.- % 3-cis- und 0,2 % 2-trans-pentensäureester zusammensetzt.

Die Katalysatorproduktivität für 4-Pentensäuremethylester beträgt 1 600 g pro g Pd und Stunde.

Nach Abtrennen des Katalysators wird das Reaktionsgemisch in einer Kolonne (Trennstufenzahl 100, Rücklauf 50) aufdestilliert. Dabei werden bei einer Kopftemperatur von 126 °C, 8,2 g 4-Pentensäuremethylester mit einer Reinheit 95 % abgenommen. Der Destillationssumpf (91,8 g) wird nach Ergänzen der abdestillierten Menge (8,2 g) mit frischem Pentensäureestergemisch der anfangs genannten Zusammensetzung erneut zur Isomerisierung eingesetzt.

Nach 30 Recyclisierungen hat die Aktivität des Katalysators soweit abgenommen, daß man nach 6 Minuten Reaktionszeit ein Reaktionsgemisch erhält, welches noch 6,4 Gew.- % 4-Pentensäureester enthält. Der Katalysator wird nach dem Abtrennen vom Reaktionsgemisch mit Methanol gewaschen, zum Entfernen des Methanols evakuiert und dann bei 400 bis 450 C mit Luft abgebrannt. Nach dieser Behandlung ist die Aktivität des Katalysators wieder voll hergestellt.

**Patentansprüche**

1. Verfahren zur Herstellung von 4-Pentensäureestern durch Isomerisierung von Pentensäureestern bei erhöhter Temperatur mit Katalysatoren, dadurch gekennzeichnet, daß man Pentensäureester mit sauren Ionenaustauschern oder sauren Zeolithen, die einen Gehalt an Edelmetallen der achten Gruppe des periodischen Systems haben, behandelt und 4-Pentensäureester aus dem Reaktionsgemisch abdestilliert.

2. Verfahren nach den Anspruch 1, dadurch gekennzeichnet, daß die sauren Ionenaustauscher oder sauren Zeolithe ein Gehalt an Palladium, Ruthenium oder Rhodium haben.

3. Verfahren nach den Ansprüchen 1 und 2, dadurch gekennzeichnet, daß die sauren Ionenaustauscher oder sauren Zeolithe einen Gehalt von 0,01 bis 1 Gew.- % an Edelmetallen der achten Gruppe des periodischen Systems haben.

4. Verfahren nach den Ansprüchen 1 bis 4, dadurch gekennzeichnet, daß man eine Temperatur von 100 bis 150 °C einhält.

5. Verfahren nach den Ansprüchen 1 bis 5, dadurch gekennzeichnet, daß man eine Verweilzeit von 0,01 bis 1 Stunden am Katalysator einhält.

**Claims**

1. A process for the preparation of a 4-pentenoate by isomerization of pentenoates at elevated temperature with a catalyst, wherein pentenoates are treated with an acidic ion exchanger or acidic zeolite which contains a noble metal of group eight of the periodic table, and the 4-pentenoate is distilled off from the reaction mixture.

2. A process as claimed in claim 1, wherein the acidic ion exchanger or acidic zeolite contains palladium, ruthenium or rhodium.

3. A process as claimed in claims 1 and 2, wherein the acidic ion exchanger or acidic zeolite contains from 0.01 to 1 % by weight of a noble metal of group eight of the periodic table.

4. A process as claimed in claims 1 to 3, wherein a temperature of from 100 to 150 °C is maintained.

5. A process as claimed in claims 1 to 4, wherein the residence time over the catalyst is from 0.01 to 1 hour.

**Revendications**

1. Procédé de préparation d'esters 4-penténiques par isomérisation d'esters penténiques à température élevée, avec des catalyseurs, caractérisé par le fait que l'on traite des esters penténiques avec des échangeurs d'ions acides ou des zéolithes acides, qui contiennent un métal noble du huitième groupe du système périodique, et on distille les esters 4-penténiques du mélange de réaction.

2. Procédé selon la revendication 1, caractérisé par le fait que les échangeurs d'ions acides ou les zéolithes acides contiennent du palladium, ruthenium ou rhodium.

3. Procédé selon l'une des revendications 1 ou 2, caractérisé par le fait que les échangeurs d'ions acides ou les zéolithes acides contiennent 0,01 à 1 % en poids de métal noble du huitième groupe du système périodique.

4. Procédé selon l'une des revendications 1 à 3, caractérisé par le fait que l'on maintient une température de 100 à 150 °C.

5. Procédé selon l'une des revendications 1 à 4, caractérisé par le fait que l'on maintient un temps de séjour du catalyseur de 0,01 à 1 heure.